Europäisches Patentamt

European Patent Office

Office européen des brevets

(19)

(11) Veröffentlichungsnummer : **0 063 097**
**B1**

(12) **EUROPÄISCHE PATENTSCHRIFT**

(45) Veröffentlichungstag der Patentschrift :
21.10.87

(21) Anmeldenummer : **82730045.0**

(22) Anmeldetag : **13.04.82**

(51) Int. Cl.⁴ : **A 61 N   1/368**

(54) **Herzschrittmacher.**

(30) Priorität : **10.04.81 DE 3115124**

(43) Veröffentlichungstag der Anmeldung :
**20.10.82 Patentblatt 82/42**

(45) Bekanntmachung des Hinweises auf die Patenterteilung : **21.10.87 Patentblatt 87/43**

(84) Benannte Vertragsstaaten :
**AT CH DE FR GB IT LI NL SE**

(56) Entgegenhaltungen :
**DE-A- 2 455 237
DE-A- 2 528 817
DE-A- 2 701 104
DE-A- 2 701 481
DE-A- 2 739 490
DE-A- 2 845 323
DE-A- 2 943 583
DE-A- 2 944 572
DE-A- 2 944 596
DE-A- 2 944 631**

(73) Patentinhaber : **BIOTRONIK Mess- und Therapiegeräte GmbH & Co Ingenieurbüro Berlin
Sieversufer 8
D-1000 Berlin 47 (DE)**

(72) Erfinder : **Schaldach, Max, Prof.Dr.-Ing.
Turnstrasse 5
D-8520 Erlangen (DE)**
Erfinder : **Keller, Walter J.
8600-S.W. 54th Avenue
Miami Florida 33 143 (US)**

(74) Vertreter : **Christiansen, Henning, Dipl.-Ing.
CHRISTIANSEN & NINNEMANN Dietrich-Schäfer-Weg 21
D-1000 Berlin 41 (DE)**

## Beschreibung

Die Erfindung betrifft eine Vorrichtung der im Oberbegriff des Anspruch 1 angegebenen Art. Eine derartige Vorrichtung ist aus der DE-A-2 701 104 bekannt.

Die Herzstimulation wird inzwischen für Herzblöcke verschiedener Grade und besondere weitere Arrhythmien allgemein klinisch und therapeutisch anerkannt. Mit der sogenannten physiologischen Stimulation hat sich dabei eine Methode herausgebildet, bei der nicht nut entweder der Ventrikel oder das Atrium, sondern beide Kammern in einer Weise stimuliert werden, die den natürlichen Gegebenheiten entgegenkommt. Samet und Castillo haben die sogenannte « Sequentielle Stimulation » im Zusammenhang mit einer Studie der hämodynamischen Werte des Herzens für einen weiten Bereich von Stimulationsparametern verwirklicht (Samet, B., Castillo, C., Bernstein, W. H. « Hemodynamic Sequelae of atrial, ventricular and sequential atrial ventricular Pacing in cardiac Patients », American Heart Journal, Bd. 72, Seiten 725 bis 729, Dezember 1966).

Schon früher wurden durch eine P-Wellen-synchrone Kopplung der Stimulation beider Kammern wesentlich bessere antiarrhythmische Eigenschaften erzielt als beim Stimulieren ausschließlich im Ventrikel (Nathan, D. A., Center, S., Wu, C. Y., Keller, W., « An implantable synchronous Pacemaker for the long Term Correction of complete Heart Block », American Journal of Cardiology, Bd. 11, Seite 362, 1963).

Bradykardien wurden dabei mit einem entsprechenden ventrikulären Rhythmus behandelt, der mit der tatsächlichen Sinusrate in Konkurrenz trat oder mit dieser außer Synchronität war.

Die implantierbaren Versionen von AV-sequentiellen Schrittmachern, wie sie in der US-PS 3 747 604 dargestellt sind und auf den Arbeiten von Samet und Castillo beruhen, weisen eingeprägte Escape-Intervalle für atriale Impulse vor denjenigen für ventrikuläre Impulse auf, so daß der arrhythmische Schutz insbesondere im bradykardem Bereich heraufgesetzt ist. (Der Begriff « Escape-Intervall » bedeutet in diesem Zusammenhang, daß der künstliche Herzschrittmacher einen Stimulationsimpuls abgibt, wenn nicht innerhalb eines vorgegebenen Intervalls ein entsprechendes herzeigenes Signal erscheint.)

Die Arrhythmiestudien und Beobachtungen von Castellanos (Castellanos, A. ; Waxmann, H. L., Moleiro, F., Berkovitz, F. V. und Lung R. J. « Preliminary Studies with an implantable multimode A-V Pacemaker for reciprocrating atrioventricular Tachycardias », Bd. 3, No. 3, Pace, Mai 1980), zeigen, auf welche Weise Arrhythmien durch geeignete Stimulation einer der beiden Herzkammern eingeleitet, beseitigt und unterdrückt werden können, selbst wenn die Kopplung für einige Patienten in umgekehrter Richtung erfolgt.

Die Literatur über kardiale Arrhythmien ist voll von Diagrammen und Aufstellungen über Kriterien für die « Reentry-Arrhythmien » vom dominanten Typ, wobei zwei Reizleitungswege vorhanden sein müssen, um die Arrhythmie aufrechtzuerhalten. Es ist dabei gleichzeitig dargestellt, daß die Verzögerungszeit der Schleife relativ lang sein muß, um die Bedingungen für die Aufrechterhaltung des Wiedereintritts zu erfüllen. Alle bisherigen Versuche, Tachyarrhythmien durch Stimulation zu beenden, weisen den Nachteil auf, daß die Erfolgsquote bei den verschiedenen Methoden für sich genommen noch nicht befriedigend ist.

Der in Anspruch 1 angegebenen Erfindung liegt die Aufgabe zugrunde, einen Herzschrittmacher der eingangs genannten Gattung anzugeben, welcher verbesserte Möglichkeiten bietet, derartige Reentry-Arrhythmien zu beenden.

Die Erfindung beruht auf der Erkenntnis, daß mehrere Wege der Steuerung beschritten werden müssen, um der normalen und anormalen physiologischen Verzögerungen Herr zu werden, welche das Herz veranlassen, unerwünscht hohe Raten anzunehmen.

Es hatte sich zwar einerseits bei einigen Patienten gezeigt, daß kürzere AV-Verzögerungen sich als vorteilhaft erweisen, wenn es darum geht, einen arrhythmischen Zustand zu beenden. Bei anderen Patienten wiederum war es die retrograde Stimulation des Atriums, die günstigere Ergebnisse brachte. Bis jetzt wurde die Idee der Benutzung beider Wege in einer einzigen Einheit und gleichzeitig jedoch noch nicht untersucht, obgleich eine der Voraussetzungen für derartige Arrhythmien, die seit langer Zeit bekannt ist, die Existenz zweier Wege ist, von denen mindestens einer eine relativ große Übergangsverzögerung aufweisen muß.

Vorteilhaft bei der Erfindung ist, daß bei der Anwendung der beanspruchten Mittel die Zahl derjenigen Arrhythmie-Patienten wesentlich vergrößert wird, welche auf eine derartige Stimulationstherapie zur Unterdrückung von Tachykardien ansprechen. Deren absolute Zahl ist dabei größer als allein durch die Summierung der beiden Maßnahmen erwartet werden könnte, da beide therapeutische Maßnahmen sich gegenseitig ergänzen und ein Beenden einer Tachyarrhythmie auch dann herbeiführen können, wenn eine der beiden Maßnahmen allein dazu nicht ausgereicht hätte.

Die Kriterien, die im angeführten Stand der Technik genannt sind, geben das Verhältnis der Ausbreitungszeit über natürliche und zusätzliche Wege zum Anregungsintervall und das Verhältnis der Refraktärzeit zum Anregungsintervall wieder. Diese relativen Werte sind wichtige Faktoren, welche den Beginn oder die Aufrechterhaltung der Tachykardie hervorrufen oder begünstigen.

Es ist aus der angegebenen Literatur ersichtlich, daß die Ausbreitungszeit von Reizsignalen sich im Verhältnis zur Geweberefraktärzeit verlängern kann, wenn die Anregungszyklen verkürzt werden. Wenn eine frühe Gewebeanregung irgendwo im Verlauf der Schleife sich in einer Richtung als refraktär erweist und nach der Fortsetzung des Umlaufs auf dem anderen Weg relativ langsam die Schleife durchläuft, wird sie beim weiteren Voranschreiten das gesamte Gewebe nicht refraktär finden. Dies ist der grundsätzliche Mechanismus einer Reentry-Arrhythmie. Der Wiedereintritt der Schleife kann durch Heraufsetzen der Refraktärzeit oder durch Herabsetzen der Ausbreitungsverzögerung irgendwo in der Schleife unterbrochen werden. Es ist in diesem Zusammenhang eine wesentliche weitere wünschenswerte Eigenschaft des erfindungsgemäßen zusätzlichen parallelen Wegs, daß sich die betreffenden Zeiten mit dem durch den Reizleitungszyklus gebildeten Intervall verkürzen oder wenigstens nicht verlängern, um auf diese Weise die oben erwähnten physiologischen Änderungen zu kompensieren.

Vorteilhaft bei dem erfindungsgemäßen Herzschrittmacher ist auch, daß durch die Erkennung und Auswahl derjenigen Teile des Herzzyklus für die Stimulation, welche durch Schrittmacherimpulse beeinflußbar sind, eine relativ große Störsicherheit in Bezug auf nicht vom Herzen ausgehende Signalereignisse erreicht werden kann.

Ein vorteilhaftes Ausführungsbeispiel der Erfindung ist in den Figuren dargestellt und wird nachfolgend einschließlich bevorzugter Weiterbildungen, die in den Unteransprüchen angegeben sind, näher beschrieben. Es zeigen :

Figur 1 ein Blockschaltbild derjenigen Elemente des erfindungsgemäßen Schrittmachers, welche zum Erzeugen von Stimulationsimpulsen und zur Aufnahme und Verarbeitung intrakardialer Signale dienen,

Figur 2 Programmiermittel und die nach der Erfindung zusätzlich erforderlichen Schaltmittel,

Figuren 3a bis g Zeitdiagramme, welche das Impulsverhalten verschiedener Baugruppen des Schrittmachers auf die in Fig. 3a dargestellten EKG-Signale hin wiedergeben, sowie

Figuren 4 bis 8 die Abgabe von Stimulationsimpulsen für Atrium und Ventrikel in Abhängigkeit von verschiedenen über die Eingangsstufen des Schrittmachers aufgenommene intrakardiale Signale in Form von Zeitdiagrammen.

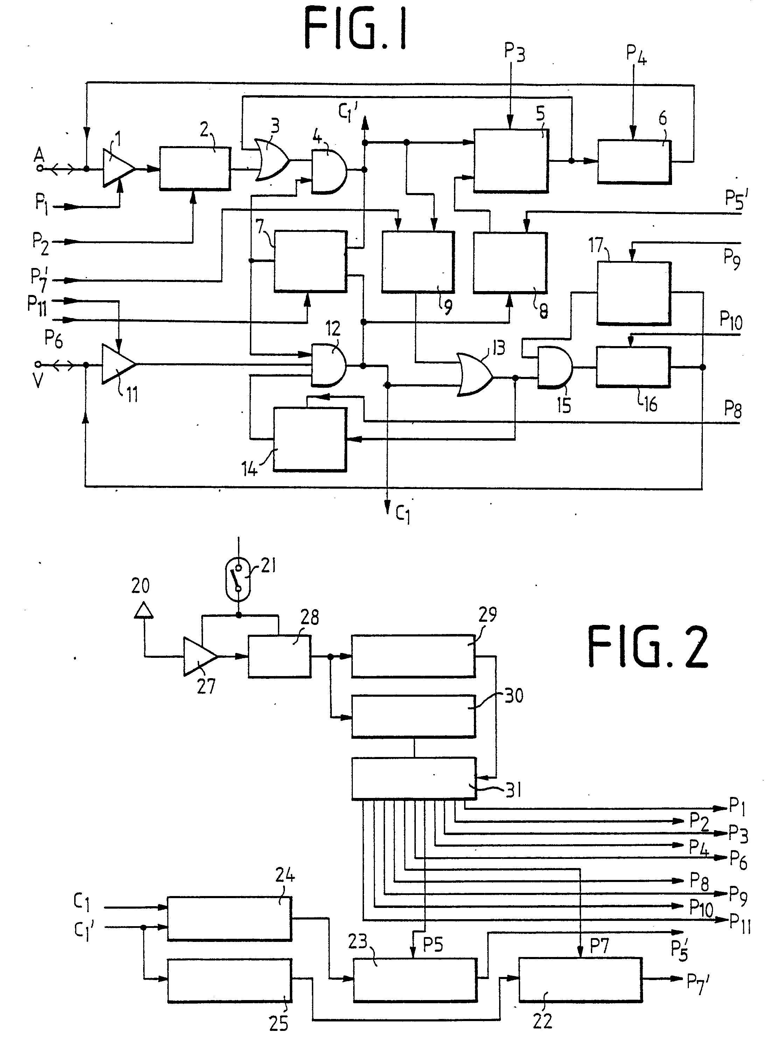

Bei dem in Figur 1 dargestellten Ausführungsbeispiel ist der Anschluß A mit einer im Atrium plazierten Elektrode verbunden. Das von der atrialen Elektrode aufgenommene Signal gelangt zu einem Vorverstärker 1, mittels dessen das Signal in seiner Amplitude so weit heraufgesetzt wird, daß es zur Ansteuerung der nachfolgenden Stufen ausreichend ist. Für die nachfolgende Beschreibung werden Signalzustände in positiver Logik (sogenannter logischer H-Zustand) im Falle der Anwesenheit von Signalen oder Impulsen vorausgesetzt.

An den Vorverstärker 1 schließt sich ein Verzögerungsglied 2 an, welches eine Verzögerung von einigen Millisekunden bewirkt. Auf den Ausgang des Verzögerungsgliedes 2 folgt ein ODER-Gatter 3, dessen Ausgang zu einem der Eingänge eines UND-Gatters 4 führt. Der Ausgang des UND-Gatters 4 ist mit dem RESET-Eingang eines Impulsgenerators 5 verbunden, welcher nach Ablauf eines vorgegebenen Zeitraums einen Ausgangsimpuls erzeugt, wenn er nicht über seinen RESET-Eingang zurückgesetzt und damit die Impulsabgabe inhibiert wird. Das Ausgangssignal des Impulsgenerators 5 steuert eine Impulsformerschaltung 6 an, in der auf einen Ausgangsimpuls des Generators 5 das Stimulationssignal für den Vorhof erzeugt wird, das an den Anschluß A gelangt, mit dem die zum Vorhof führende Elektrode verbunden ist.

Der Impulsgenerator 5 weist weiterhin einen « Sync »-Eingang auf, welcher auf einen entsprechenden Eingangsimpuls hin den Impulsgenerator synchronisiert, d. h. auf ein Eingangssignal die sofortige Abgabe eines Ausgangsimpulses veranlaßt. Auch bei einem derartigen über den « Sync »-Eingang veranlaßten Ausgangsimpuls erfolgt ein Zurücksetzen des Impulsgenerators 5 durch das von seinem Ausgang zum weiteren Eingang des ODER-Gatters 3 gelangende Signal, wenn nicht das UND-Gatter 4 dadurch gesperrt ist, daß sein weiterer Eingang auf dem sogenannten logischen L-Potential liegt. Diese Sperrung des UND-Gatters 4 ist dabei auch für die vom Ausgang des Verzögerungsgliedes 2 her zum ODER-Gatter 3 gelangenden Impulse wirksam und wird von einem (Sensing-)A-Refraktärglied 7 veranlaßt, welches an seinem Ausgang ein logisches H-Signal für eine bestimmte Zeitdauer abgibt, nachdem an einen seiner Eingänge ein Impuls gelangt ist. Dieses Refraktärglied 7 besteht bei einer möglichen Ausführung der Erfindung aus einem retriggerbaren Monoflop und sperrt damit das UND-Gatter 4 für einen Zeitraum, nachdem ein Signal vom Vorhof oder — über nachfolgend zu beschreibende weitere Schaltglieder — aus der Kammer aufgenommen wurde.

Die bisher dargestellten Bauelemente bilden einen vorhofgesteuerten Bedarfsschrittmacher, bei dem bei Unterschreitung einer vorgegebenen Herzeigenfrequenz künstliche Stimulationsimpulse abgegeben werden. Für einen vorgegebenen Zeitraum nach Abgabe des letzten künstlichen Stimulationssimpulses oder Aufnahme eines herzeigenen Signals verhält sich der Schrittmacher refraktär.

Im Rahmen eines programmierbaren Schrittmacher-Konzepts sind der Verstärkungsfaktor des Eingangsverstärker 1 über eine Leitung $P_1$, die Verzögerungszeit des Verzögerungsglieds 2 über eine Leitung $P_2$, die Eigenfrequenz des Impulsgenerators 5 beim Fehlen von RESET-Impulsen über eine Leitung $P_3$ und die Amplitude der Ausgangsimpulse des Verstärkers 6 über eine Leitung $P_4$ programmierbar.

Ein weiterer Teil des Schrittmachers wird durch einen mit dem Anschluß VI für eine im Ventrikel plazierte Elektrode verbundenen Eingangsverstärker 11 für Ventrikelsignale gebildet, dessen Ausgang mit einem UND-Gatter 12 verbunden ist, dessen Signale wiederum an ein ODER-Gatter 13 gelangen. Das Ausgangssignal des ODER-Gatters 13 steuert ein (Sensing-)Refraktär-Glied $VR_s$ 14 an, welches für einen vorgegebenen Zeitraum, der mit einem vom ODER-Gatter 13 ausgehenden Impuls beginnt, ein L-Signal abgibt, das über einen weiteren Eingang des UND-Gatters 12 letzteres während des vorgenannten vorgegebenen Zeitraums für ein im Ventrikel aufgenommenes Signal sperrt. Eine entsprechende Sperrung wird auch durch ein vom Ausgang des Refraktär-Gliedes 7 an einen weiteren Eingang des UND-Gatters 12 gelangendes L-Signal bewirkt, so daß der Leitungsweg für Ventrikelsignale auf zwei verschiedene Weisen für unterschiedlich lange Zeiträume inhibierbar ist. Eine Aktivierung des Refraktär-Gliedes 7 findet dabei auch über vom Eingang des Atrium-Vorverstärkers 1 ausgehende Signale statt.

Das $VR_s$-Refraktär-Glied 14 sperrt die Auslösung von Ventrikel-Stimulationsimpulsen dann, wenn vom ODER-Gatter 13 Signale abgegeben werden, d. h., wenn bei nicht gesperrtem UND-Gatter 12 am Anschluß V Signale aufgenommen wurden, für einen Zeitraum von 400 ms.

Das Ausgangssignal des ODER-Gatters 13 gelangt zusätzlich an ein UND-Gatter 15, welches eine Ausgangsstufe 16 als Impulsformerstufe zur Erzeugung der den Ventrikel stimulierenden Signale ansteuert. Der Ausgang der Stufe 16 ist mit dem Anschluß für die Ventrikelelektrode V und damit gleichzeitig mit dem Eingangsverstärker 11 verbunden.

Mittels von der Ausgangsstufe 16 erzeugte Impulse wird ein zusätzliches (Ausgangs-)Refraktär-Glied $VR_0$ 17 angesteuert, welches auf diese Impulse hin für eine vorgegebene Zeitdauer ein L-Signal erzeugt, das das UND-Gatter 15 für weitere vom ODER-Gatter 13 her erscheinende Signalimpulse sperrt.

Das $VR_0$-Refraktärglied 17 bildet dabei ein Zeitglied für das ventrikuläre Ausgangssignal. Es dient dazu, die Rate der ventrikulären Stimulation zu begrenzen. Falls die Sinus-Rate die Tendenz hat, eine zu kurze Impulsfolge des Schrittmachers zu veranlassen, so bildet die Zeit $VR_0$ von 400 ms eine der Patientensicherheit dienende Ratenbegrenzung.

Der Signalweg zur Verstärkung von aus dem Ventrikel abgeleiteten Signalen bzw. zur Erzeugung von Stimulationsimpulsen für den Ventrikel besteht damit aus Mitteln, welche für sich, getriggert von herzeigenen Aktionen im Ventrikel, Stimulationsimpulse für den Ventrikel auslösen, wenn nicht die Folgezeit zwischen den Impulsen so klein ist, daß eines der Refraktär-Glieder 7, 14 oder 17 über die UND-Gatter 12 bzw. 15 die Weitergabe der Impulse an das nachfolgende Schaltungsglied unterbindet. Das Refraktär-Glied 7 sperrt damit auf ein im Atrium oder im Ventrikel aufgenommenes Signal die Weiterverarbeitung von Impulsen in beiden Signalwegen, wobei im Verarbeitungsweg für Atriumsignale der Impulsgenerator 5 inhibiert wird, so daß hier die Abgabe von Ausgangsimpulsen unterdrückt und im Signalweg für vom Ventrikel aufgenommene Signale die Erzeugung von Stimulationsimpulsen im Ventrikel unterbunden wird, welche möglicherweise in die vulnerable Phase des Herzens fallen könnten.

Ein (VA-)Verzögerungsglied 8 gibt ein im Normalfall um 25 ms verzögertes Signal ab, wenn vom UND-Gatter 12 an seinen Eingang ein Impuls gelangt, d. h. vom Eingangsverstärker 11 ein Signal abgegeben wird, ohne daß das UND-Gatter 12 über die Refraktär-Glieder 7 oder 14 gesperrt ist. Ein derartiger Signalimpuls rührt auf Grund der gewählten Refraktärzeiten von einer vorzeitigen ventrikulären Kontraktion her.

Das Ausgangssignal des Verzögerungsgliedes 8 gelangt an den « SYNC »-Eingang des Impulsgenerators 5 und bewirkt damit eine sofortige Impulsabgabe durch die Impulsausgangsstufe 6 des atrialen Signalwegs, wodurch der Impulsgenerator 5 auch zurückgesetzt wird. Das Ausgangssignal des UND-Gatters 12 gelangt gleichzeitig an das Refraktär-Glied 7, woraufhin die Abgabe des die atriale Refraktärzeit bestimmenden Impulses veranlaßt wird. Die Verzögerungszeit des (VA-)Verzögerungsgliedes ist sehr kurz gewählt, so daß der nachfolgende Stimulationsimpuls für das Atrium nahezu unmittelbar darauffolgend abgegeben wird.

Ein weiteres Verzögerungsglied 9, dessen Verzögerungsdauer im normalen Stimulationsbetrieb der physiologischen AV-Überleitungszeit von ca. 170 ms entspricht, ist mit dem Ausgang des UND-Gatters 4 verbunden und gibt auf einen Eingangsimpuls ein verzögertes Ausgangssignal ab. Dieser Eingangsimpuls hat auch den Impulsgenerator 5 zurückgesetzt, d. h. er wurde als außerhalb der Refraktärzeit des Atriums erscheinender Impuls verarbeitet. Das Ausgangssignal des Verzögerungsgliedes 9 gelangt zu einem weiteren Eingang des ODER-Gatters 13, welcher über das UND-Gatter 15 die Ausgangsstufe für den Ventrikel ansteuert und damit einen Stimulationsimpuls für den Ventrikel veranlaßt.

Die Verzögerungsglieder 8 und 9 sind bezüglich der Verzögerungszeiten über Leitungen $P_7$, bzw. $P_5$, hinsichtlich der Verzögerungsdauer durch Steuermittel, welche im Anschluß beschrieben werden, veränderlich.

Über eine weitere Programmierleitung $P_9$ ist das Refraktärglied $VR_0$ 17, und über Programmierleitungen $P_{11}$, $P_8$ und $P_{10}$ der Eingangsverstärker 11, das Refraktärglied $VR_s$ 14 bzw. die Amplitude der von der Ausgangsstufe 16 abgegebenen Impulse zu beeinflussen.

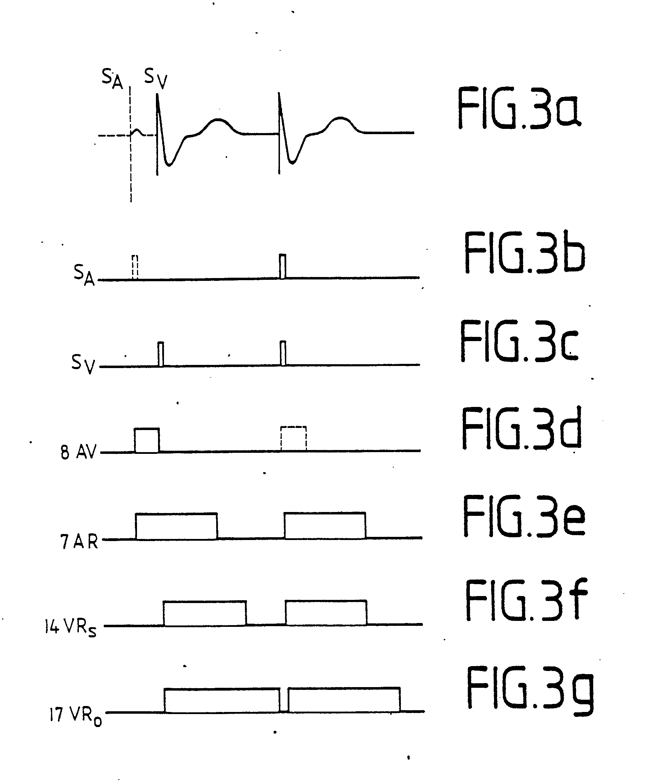

Der Betrieb des bisher beschriebenen Schrittmacherteils soll nunmehr anhand der Figuren 3a bis g erläutert werden.

In Figur 3a ist der Verlauf des zugehörigen EKG-Signals dargestellt, wobei Impulse $S_A$ Stimulationsimpulse im Atrium und $S_V$ Stimulationsimpulse im Ventrikel bedeuten (Figuren 3b und c). Mit AV ist die Überleitungszeit vom Atrium zum Ventrikel bezeichnet (Figur 3d). Die Refraktärzeit AR für das Atrium (Refraktärglied 7 in Figur 1) und die beiden Refraktärzeiten $VR_s$ und $VR_0$ für den Ventrikel (Refraktärglieder 14 und 17 in Figur 1) sind in den Figuren 3e, f bzw. g wiedergegeben.

Es ist ersichtlich, daß die sich für den Ventrikel ergebende Gesamtrefraktärzeit für Eingangssignale (Sensing) sich aus der Summe von AV-Verzögerung und Refraktärzeit des Glieds 14 VR$_s$ zusammensetzt.

Die Refraktärzeiten — wie sie in den Figuren 3e bis g in Abhängigkeit von in Atrium und Ventrikel auftretenden Impulsen wiedergegeben sind — bestimmen maßgeblich die Möglichkeiten des Schrittmachers, durch Stimulation im Sinne einer Terminierung von Tachyarrhythmien wirksam zu werden. Die gestrichelten Impulse in Figur 3 sind nicht immer vorhanden und hängen vom Betriebszustand des Schrittmachers bzw. vom vorhergehenden Herzverhalten ab. So erscheint der Impuls S$_A$ nur dann, wenn im Atrium innerhalb eines durch die Refraktärzeiten vorgegebenen Zeitfensters kein Spontanimpuls erscheint.

Zum Betrieb des zuvor beschriebenen Schrittmachers sind noch die folgenden Bemerkungen von Bedeutung :

Das AV-Verzögerungsglied 8 ist programmierbar ausgebildet. Da der Ventrikel-Impulsgenerator niemals inhibiert wird — es sei denn, es liegt ein partieller Block vor, besteht bei dem dargestellten Ausführungsbeispiel kein Bedarf für Störungsunterdrückungsmittel. Weiterhin ergibt sich eine wichtige Unterscheidung zwischen den atrialen und ventrikulären Impulsausgangs-Funktionen. Das atriale System erzeugt Impulse, welche vom Escape-Intervall unabhängig sind. Der Ventrikelimpuls ist entweder von atrialen, ventrikulären oder Störungsereignissen abhängig und erfolgt niemals spontan.

Weil die simultane Detektion einer ventrikulären Systole auf beiden am Herzen angebrachten Elektroden eines unipolaren Leitersystems auftreten kann, erzeugt das Verzögerungsglied 2 eine kurze Verzögerung von einigen Millisekunden, um sicherzustellen, daß dieses Signal als Ventrikelsignal ausgewertet und nicht als Ereignis im Vorhof interpretiert wird. Mit der Verzögerung des Glieds 2 sperrt eine normale P-Welle den Eingang für Ventrikelimpulse und leitet mit der geringfügigen Verzögerung einen neuen Zyklus ein. Falls es sich bei dem ersten Spontanimpuls dieses Zyklus um eine vorzeitige Kammerkontraktion handelt, ermöglicht die kurze Verzögerung in der atrialen Verarbeitung der ventrikulären Eingangsschaltung fortzufahren und das Verhalten zu kontrollieren.

Diese Verzögerung war bei früheren sequentiellen Systemen nicht notwendig, da vorzeitige Impulse in Ein-Richtungs-Systemen nicht unterschieden werden mußten.

Im Anschluß an das Durchlaufen des UND-Gatters 4 löst das atriale Signal eine atriale Refraktärzeit aus, wie sie durch das Refraktärglied 7 repräsentiert ist. Unabhängig davon, ob das atriale Ereignis spontan oder stimuliert auftrat, sorgt das Refraktärglied 7 mit seiner Refraktärfunktion dafür, daß sowohl die atriale als auch die ventrikuläre Signalaufnahme für die Gatter 4 bzw. 12 gesperrt wird.

Es ist weiterhin zu beachten, daß der atriale Impulsgenerator 5 auf zwei verschiedene Weisen reagieren kann. Im Falle eines atrial aufgenommenen Herzschlags wird er in den Anfangszustand gesetzt nach Art eines Oszillators, der bei fehlenden Impulsen ein Signal erzeugt. Der Oszillator 5 wird an einen Anfangspunkt des Zyklus zurückgesetzt, an dessen Ende ein Stimulus erzeugt wird. Wie bei einem ventrikulär aufgenommenen Ereignis wird der atriale Impulsgenerator 5 über seinen Sync-Anschluß zeitlich vorangesetzt, welcher bewirkt, daß der Oszillator mit dem Ende des Zyklus synchronisiert wird und einen atrialen Stimulus abgibt, bevor ein neuer Zyklus eingeleitet wird.

Das AV-Verzögerungsglied 9 erzeugt ein programmierbares Zeitintervall zwischen einem ventrikulär festgestellten Ereignis und dem durch retrograde Überleitung erzeugten Stimulus.

Im Normalfall, wenn die atriale Systole zuerst auftritt und sowohl die atriale als auch die ventrikuläre Signalaufnahme blockiert, wird das einen Zeitgeber bildende AV-Verzögerungsglied 9 gestartet und auf das Ende des erzeugten Signalimpulses hin ein Triggersignal als ventrikulärer Ausgangsimpuls (Ausgangsstufe 16) generiert, nachdem das ODER-Gatter 13 und das UND-Gatter 15 von dem Impuls passiert wurden.

In Figur 2 sind in Form einer Blockschaltung zusätzliche Baugruppen dargestellt, welche die Funktion der in Figur 1 dargestellten Schrittmacherbaugruppen ergänzen. Dazu gehörig sind einerseits die Mittel, welche im Sinne einer Programmierung gestatten, verschiedene Parameter des Schrittmachers zu beeinflussen, wie sie weiter oben aufgeführt wurden. Ein über eine im Schrittmacher vorgesehene Antenne 20 übertragenes, die Betriebsparameter des Schrittmachers in verschlüsselter Form enthaltendes Signal wird bei zusätzlicher Betätigung eines Reed-Schalters 21 mittels eines Magneten über einen Eingangsverstärker 27 zu einem Detektor 28 übertragen, welcher die eingegebenen Parameter nach einer durch eine Prüfschaltung 29 ausgeführten Plausibilitäts- oder ähnlichen Kontrolle in ein Programmregister 30 einliest, wobei über ein Latch 31 die einzelnen Betriebsparameter des Schrittmachers entsprechend der Programmierung gesetzt werden.

Die Programmsignale für die VA-Verzögerung P$_{5'}$ und die Verzögerung für die AV-Verzögerung P$_{7'}$ werden durch weitere Schaltmittel beeinflußt, welche von den ermittelten Herzsignalen abhängen. Ein vom Ventrikel aufgenommenes Signal, das vom UND-Gatter 12 durchgelassen wird, weil es in keine der Refraktärzeiten für ventrikuläre Signale fällt, d. h. eine Extrasystole darstellt, gelangt vom Ausgang C$_1$ in Figur 2 zum entsprechenden Eingang der Schaltung in Figur 2 und taktet dort einen Zähler für vorzeitige Ventrikel-Signale 24, der damit eine Steuerschaltung für die VA-Verzögerung 23 — ausgehend von einem Minimalwert von 25 ms — jeweils um eine Stufe von 12,2 ms bis zu einem Maximalwert von 87,5 ms heraufsetzt. Dieses stufenweise Heraufsetzen erfolgt durch jedes weitere entsprechende Signal vom Ventrikel und erzeugt damit — ausgehend von einem über äußere Programmiermittel eingestellten Steuersignal für die VA-Verzögerung (Leitung $P_5$) — ein Ausgangssignal $P_5$, welches an das (VA-)Verzögerungsglied 8 in Figur 1 gelangt und die wirksame Einstellung des VA-Verzögerungsglieds 8 veranlaßt.

Zurückgesetzt wird der Zähler 24, wenn am Ausgang des UND-Gatters 4 ein Impuls erscheint, d. h. außerhalb der Atrium-Refraktärzeit ein Signal im Atrium festgestellt wurde (Signal $C_1$, in Figuren 1 und 2). Durch das letztgenannte Signal wird gleichzeitig ein Intervalldetektor 25 angesteuert, welcher ein Ausgangssignal abgibt, wenn die Folge zweier Atriumsignale den vorgegebenen Zeitabstand von 380 ms unterschreitet. Die Mittel können aber bei einer anderen bevorzugten — nicht dargestellten — Ausführungsform der Erfindung auch entsprechend ansprechen, wenn die vom Herzen aufgenommene Durchschnittssinusrate innerhalb eines vorgegebenen weiteren Zeitraums einen Grenzwert überschreitet. Das Ausgangssignal des Intervalldetektors 25 aktiviert eine Umschaltvorrichtung 22 für die AV-Verzögerung, welche die mittels des Signals $P_7$ programmierte Verzögerung verringert (Ausgangssignal $P_7$). Diese Verringerung wird aufgehoben, wenn sich die Sinusrate wieder auf einen normalen Wert einstellt oder das Intervall zwischen zwei herzeigenen Atriumimpulsen erneut seinen Normalwert erreicht hat.

Der Wert der AV-Verzögerung des Verzögerungsglieds 9 ist damit nicht nur vom Schrittmacher-Programmspeicher her programmier- oder einstellbar, sondern dieser wird automatisch über die Zeitmeßschaltung von Block 25 u. 22 in Fig. 2 von 170 auf 85 ms verkürzt, wenn sich Sinusintervalle ergeben, welche einen vorgegebenen Wert unterschreiten. Die Intervalle für die AV-Verzögerung lassen sich ebenso wie diejenigen der VA-Verzögerung zusätzlich über die genannten Programmiermittel von außerhalb des Körpers her beeinflussen.

Entsprechend kann die VA-Verzögerungzeit des Verzögerungsglieds 8 über das Programm im Speicher 30 von Fig. 2 voreingestellt werden und wird automatisch über die Steuerschaltung 23 bei jedem nachfolgenden ventrikulären Ereignis heraufgesetzt, das schrittmachend wirkt. Der Zähler 24 in Fig. 2, wird von einem Signal auf Null gesetzt, welches vom UND-Gatter 4 in Fig. 1 stammt und erscheint, wenn ein erster vom Atrium stammender Impuls entweder spontan oder als Demandimpuls aufgetreten ist.

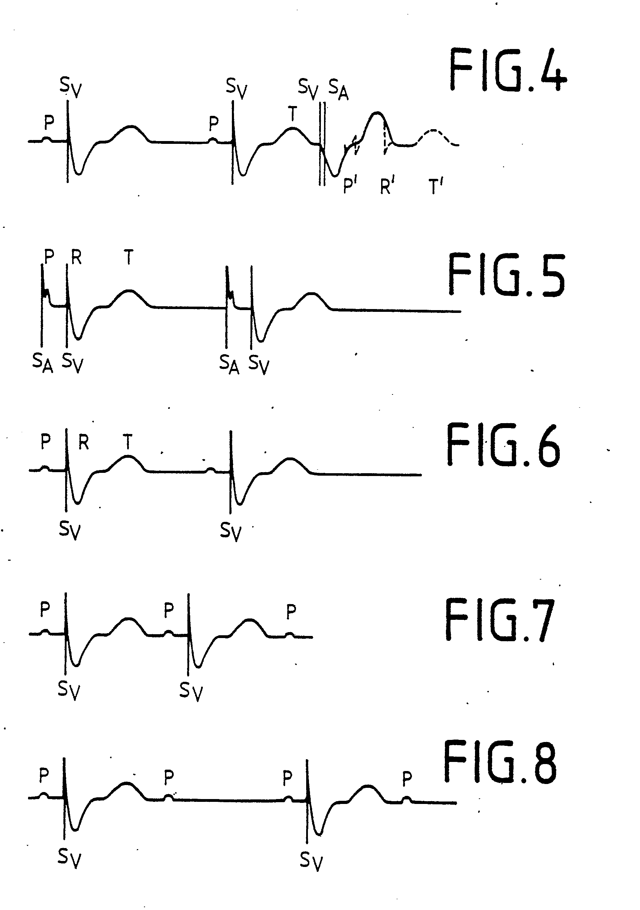

Wenn das ventrikuläre Ausgangssignal durch ein einer vorzeitigen Kontraktion im Ventrikel entsprechendes Signal blockiert ist, so ist es im Sinne der Erfindung wünschenswert, daß dieses zu einer vorzeitigen Ventrikelkontraktion gehörige Signal aufgenommen und verarbeitet wird, d. h. es wird ein atrialer Stimulus erzeugt, um jeden zusätzlichen Weg im Herzen zu blockieren und den physiologischen Zeitgeber zurückzusetzen, um einen neuen Herzzyklus zu beginnen und sicherzustellen, daß der nächste übergeleitete Herzschlag nicht in die vulnerable Phase eines vorzeitigen ventrikulären Herzschlags fällt. Fig. 4 zeigt diesen Vorgang an einem als Beispiel dargestellten EKG-Verlauf.

Es soll jetzt betrachtet werden, wie Signale zu verarbeiten sind, die von dem ventrikulären Eingangssystem sehr frühzeitig festgestellt werden. Offensichtlich kann jedes vor der ventrikulären Erholzeit erscheinende Signal nicht ventrikulärer Herkunft sein und sollte auf den Schrittmacher ohne Einfluß bleiben. Derartige störende Signale (Elektroden-Artefakte, chemische Depolarisation nach dem Stimulus, die T-Welle oder eine elektromagnetische Störung) werden durch die Refraktärzeit der Ventrikel-Eingangsschaltung unterdrückt. Außerdem wird dafür gesorgt, daß atriale Stimulussignale oder atriale spontane Aktivitäten den programmierten ventrikulären Stimulus in jedem Fall sicherstellen, wobei die Aufnahme von ventrikulären Signalen für denjenigen Zeitraum überflüssig ist, der mit der P-Welle des EKG beginnt und kurz vor der T-Welle endet. Die ventrikuläre Refraktärzeit erstreckt sich damit entweder vom Beginn eines atrialen oder eines ventrikulären Ereignisses bis zum Ende der Refraktärzeit $VR_s$.

Es ist für den auf dem Gebiet tätigen Fachmann ersichtlich, daß alle diese Parameter ohne operativen Eingriff modifizierbar sein sollten. Die Parameterwerte, welche durch das Programmspeicher-Register 30 des Systems (Fig. 2) gesteuert werden, sind in ihrem Wert dargestellt. Der Verstärker 27 für den Signalträger (HF, magnetisch, optisch etc.). Der Detektor 28 bildet einen Empfänger für die Signalmittel zu Änderung oder Einstellung des Programms in Register 30, welcher durch ein Latch 31 verriegelt ist, das die Erfüllung der Kriterien des Codes gemäß Prüfschaltung 29 überprüft.

In der nachfolgenden Beschreibung werden anhand der Figuren 4 bis 8 einige Betriebsweisen dargestellt, welche für den erfindungsgemäßen Schrittmacher typisch sind.

1. Unter der Annahme, daß die Sinusrate einen Wert von nur 30 Schlägen/min (Sinus-Bradycardie) aufweist, erzeugt der Schrittmacher jeweils einen atrialen Stimulus, der von einem ventrikulären Stimulus mit einer durch das Escape-Intervall vorgegebenen Rate und einer vorgegebenen Zeitverzögerung zwischen A und V gefolgt wird, wie es in Fig. 5 dargestellt ist.

2. Wenn die Sinusrate größer ist als die Rate des Schrittmachers (beispielsweise 85 Herzschläge/min) setzt der Schrittmacher den atrialen Impulsgenerator jeweils zurück, ohne daß ein atrialer Stimulus abgegeben wird. Der ventrikuläre Stimulus wird bedarfsunabhängig nach dem Ablauf des vorgegebenen Zeitintervalls erzeugt. Vorausgesetzt dabei ist eine Eins-zu-Eins-Überleitung vom Atrium zum Ventrikel und eine relativ normale Herztätigkeit mit oder ohne physiologischer Überleitung, wie es in Fig. 6 dargestellt ist.

3. Wenn die Sinusrate einen vorgegebenen Wert von beispielsweise 95 Herzschlägen/min überschreitet, erzeugt der Schrittmacher ventrikuläre Impulse, die synchron mit dem atrialen her-

zeigenen Signal aber mit einem vorgegebenen verkürzten AV-Verzögerungsintervall (wie es in Fig. 7 dargestellt ist) abgegeben werden. Diese Verkürzung der AV-Verzögerung geht automatisch mit einer nicht-refraktären, vorzeitigen atrialen Kontraktion einher.

4. Unter der Annahme, daß die Sinusrate höher ist als ein vorgegebener Maximalwert für die Herzrate, von beispielsweise 160 Herzschlägen/min, wird ein ventrikulärer Impuls synchron (zeitgleich oder nahezu zeitgleich) mit einem Impuls im Atrium abgegeben. Die dabei verkürzte AV-Verzögerung bewirkt eine AV-Überleitung im Verhältnis von eins pro zwei Schläge, wobei der Ventrikel dann eine Rate von 80 Herzschlägen aufweist, wie es in Fig. 8 dargestellt ist. Medizinisch handelt es sich dabei um einen Block zweiten Grades.

5. Wenn wie vorher die Ventrikel-Kontraktion nicht vorzeitig refraktär auftritt, sondern lediglich vor dem nächsten atrialen Escape-Zeitpunkt, gibt der Schrittmacher sowohl ventrikuläre als auch atriale Impulse mit einer vorgegebenen VA-Verzögerung ab, wobei diese Verzögerungszeit beim mehrfachen Auftreten vergrößert wird und der erste vom Sinusknoten herrührende Zyklus eine Rückstellung in den Anfangszustand bewirkt. Dieser Ablauf ist in Fig. 4 dargestellt.

Die vorzeitige ventrikuläre Kontraktion löst damit einen unverzüglichen atrialen Stimulus aus. Falls der nächste Herzschlag ordnungsgemäß vom Atrium hervorgerufen wird, wird die Abtastfunktion unterbrochen und inaktiv gemacht bis wiederum zwei oder mehr Herzschläge ohne Sinuskontrolle abgelaufen sind. Das Abtasten wird fortgesetzt bis zu einer eigenen Sinus-Aktivität oder einer offensichtlichen Sinus-Aktivität bzw. bis ein vorgegebener Zählerstand erreicht ist.

Die Ausführung der vorgenannten Baugruppen erfolgt entsprechend denjenigen bekannter Schrittmacher, bei digital arbeitenden Schaltungen mit diskreten oder programmierten Logikschaltungen, wobei auch die analog arbeitenden Schaltungen (Eingangsverstärker, Ausgangsimpulsformer) jeweils für sich bereits bekannten Schaltungsbaugruppen entsprechen.

**Patentansprüche**

1. Herzschrittmacher mit Mitteln (6, 16) zur Stimulation des Atriums und des Ventrikels jeweils in vorgegebenen Zeitabständen nach einem in der jeweils anderen Herzkammer aufgenommenen Signal oder einem an die jeweils andere Herzkammer abgegebenen Stimulationsimpuls, mit ersten Zeitgebermitteln (9), welche, auf ein im Atrium aufgenommenes Signal hin nach einem gegenüber der physiologischen AV-Überleitungszeit verkürzten Verzögerungszeitraum einen Stimulationsimpuls im Ventrikel auslösen, sowie mit zweiten Zeitgebermitteln (8), welche, auf ein im Ventrikel aufgenommenes Signal hin nach einem gegenüber dem physiologischen VA-Zeitabstand verkürzten Verzögerungszeitraum einen Stimulationsimpuls im Atrium auslösen, dadurch gekennzeichnet, daß zum Beenden von Tachyarrhythmien Detektoren für vorzeitige Sinusaktivitäten (35) und/oder für Extrasystolen (34) vorgesehen sind, welche Ausgangssignale zur Aktivierung der ersten und zweiten Zeitgebermittel abgeben.

2. Herzschrittmacher nach Anspruch 1, dadurch gekennzeichnet, daß der zur Aktivierung der ersten Zeitgebermittel dienende Detektor (35) für vorzeitige Sinusaktivitäten ein Detektor zur Ermittlung des Intervalls zwischen aufeinanderfolgenden aus dem Atrium herrührenden P-Wellen ist.

3. Herzschrittmacher nach einem der vorangehenden Ansprüche, dadurch gekennzeichnet, daß die zweiten Zeitgebermittel (8) durch den Detektor für Extrasystolen (34) aktivierbar sind.

4. Herzschrittmacher nach Anspruch 3, dadurch gekennzeichnet, daß der verkürzte Verzögerungszeitraum der zweiten Zeitgebermittel bei einer ersten Extrasystole auf einen minimalen Wert einstellbar und bei jeder weiteren Extrasystole um einen vorgebbaren Wert heraufsetzbar ist.

5. Herzschrittmacher nach einem der Ansprüche 3 oder 4, dadurch gekennzeichnet, daß der Detektor für Extrasystolen ein Zähler (34) ist.

6. Herzschrittmacher nach Anspruch 5, dadurch gekennzeichnet, daß der Zähler (34) durch ein aus dem Atrium herrührendes Signal in seinen Anfangszustand zurücksetzbar ist.

7. Herzschrittmacher nach einem der vorangehenden Ansprüche, dadurch gekennzeichnet, daß mindestens ein Refraktärglied (7, 14) vorgesehen ist, welches auf ein im Atrium und/oder Ventrikel aufgenommenes Signal hin die Weitergabe weiterer im Atrium bzw. Ventrikel aufgenommenen Signale für einen vorgegebenen Zeitraum verhindert.

8. Herzschrittmacher nach Anspruch 7, dadurch gekennzeichnet, daß das Refraktärglied (7) derart ausgebildet ist, daß die Refraktärzeit bei einem im Atrium aufgenommenem Signal derjenigen bei einem im Ventrikel aufgenommenem Signal derart überlagerbar ist, daß sich die effektive Refraktärzeit für im Ventrikel aufgenommene Signale um die physiologische AV-Überleitungszeit verlängert.

9. Herzschrittmacher nach einem der vorangehenden Ansprüche, dadurch gekennzeichnet, daß mindestens ein Refraktärglied (17) vorgesehen ist, welches auf Ausgangssignale des Schrittmachers anspricht und den jeweiligen Ausgang und/oder den Eingang des zugeordneten Ausgangsverstärkers für einen vorbestimmten Zeitraum nach jedem Ausgangsimpuls sperrt.

10. Herzschrittmacher nach einem der vorangehenden Ansprüche, dadurch gekennzeichnet, daß den zweiten Zeitgebermitteln (8) ein Zeitgeber (5) nachgeschaltet ist, der derart ausgebildet ist, daß er von sich aus Stimulationsimpulse für das Atrium auslöst, wenn er innerhalb eines vorgegebenen Zeitraumes kein von den zweiten Zeitgebermitteln herrührendes Signal oder kein von der im Atrium plazierten Elektrode (A) herrührendes Signal aufnimmt.

11. Herzschrittmacher nach einem der vorangehenden Ansprüche, dadurch gekennzeichnet, daß der Ventrikel bei nicht aktivierten ersten Zeitgebermitteln (9) um einen der physiologischen AV-Überleitungszeit entsprechenden Zeitraum gegenüber einem vom Atrium herrührenden Signal und/oder einem Stimulationsimpuls für das Atrium verzögert stimulierbar ist.

12. Herzschrittmacher nach einem der vorangehenden Ansprüche, dadurch gekennzeichnet, daß bei im wesentlichen gleichzeitig im Atrium und Ventrikel aufgenommenen Signalen diejenigen im Ventrikel Vorrang haben und die betreffenden Signale als vom Ventrikel herrührend verarbeitet werden.

13. Herzschrittmacher nach Anspruch 12, dadurch gekennzeichnet, daß ein Verzögerungsglied (2) für die im Atrium aufgenommenen Signale vorgesehen ist.

14. Herzschrittmacher nach einem der vorangehenden Ansprüche, dadurch gekennzeichnet, daß Programmiermittel (29 bis 31) zur Veränderung der Parameter des Schrittmachers vorgesehen sind, und daß die Verzögerungszeiträume der ersten und zweiten Signalgebermittel über Programmiermittel jeweils auf einen für den Normalbetrieb ohne Tachyarrhythmien gültigen Wert einstellbar sind.

15. Herzschrittmacher nach Anspruch 14, dadurch gekennzeichnet, daß durch die Programmiermittel auch die verkürzten Verzögerungszeiträume vorgebbar sind, welche erst bei Aktivierung der ersten und zweiten Zeitgebermittel wirksam werden.

## Claims

1. A cardiac pacemaker having means (6, 16) for the stimulation of the atrium and of the ventricle each at preset intervals of time after a signal picked up in the other cardiac chamber or a stimulation impulse given to the other cardiac chamber, having first timing means (9) which, in response to a signal picked up in the atrium, cause a stimulation impulse in the ventricle after a delay period which is shortened in comparison with the physiological AV transfer time, and having second timing means (8) which, in response to a signal picked up in the ventricle, cause a stimulation impulse in the atrium after a delay period which is shortened in comparison with the physiological VA time interval, characterised in that in order to terminate tachyarrhythmias, detectors are provided for premature sinus activities (35) and/or for extrasystoles (34) and deliver output signals for the activation of the first and second timing means.

2. A cardiac pacemaker as claimed in Claim 1, characterised in that the detector (35) for premature sinus activities, serving to activate the first timing means is a detector for determining the interval between successive P-waves originating from the atrium.

3. A cardiac pacemaker as claimed in any one of the preceding Claims, characterised in that the second timing means (8) can be activated by the detector for extrasystoles (34).

4. A cardiac pacemaker as claimed in Claim 3 characterised in that the shortened delay period of the second timing means can be set to a minimum value on a first extrasystole and increased by a value which can be preset on each further extrasystole.

5. A cardiac pacemaker as claimed in any one of Claims 3 or 4, characterised in that the detector for extrasystoles is a counter (34).

6. A cardiac pacemaker as claimed in Claim 5, characterised in that the counter (34) can be reset to its initial state by a signal originating from the atrium.

7. A cardiac pacemaker as claimed in any one of the preceding Claims, characterised in that at least one refractory element (7, 14) is provided which, in response to a signal picked up in the atrium and/or ventricle, prevents the passing on of further signals picked up in the atrium or ventricle for a preset period of time.

8. A cardiac pacemaker as claimed in Claim 7, characterised in that the refractory element (7) is constructed in such a manner that the refractory time in the event of a signal picked up in the atrium can be superimposed on that in the event of a signal picked up in the ventricle in such a manner that the effective refractory time for signals picked up in the ventricle is extended by the physiological AV transfer time.

9. A cardiac pacemaker as claimed in any one of the preceding Claims, characterised in that at least one refractory element (17) is provided which responds to the output signals of the pacemaker and blocks the particular output and/or the input of the associated output amplifier for a predetermined period of time after each output pulse.

10. A cardiac pacemaker as claimed in any one of the preceding Claims, characterised in that the second timing means (8) are followed by a timing-pulse generator (5) which is constructed in such a manner that, of its own accord, it releases stimulation impulses for the atrium if, within a preset period of time, it does not receive any signal originating from the second timing means or any signal originating from the electrode (A) placed in the atrium.

11. A cardiac pacemaker as claimed in any one of the peceding Claims, characterised in that, if the first timing means (9) are not activated, the ventricle can be stimulated, delayed by a period of time corresponding to the physiological AV transfer time relative to a signal originating from the atrium and/or a stimulation impulse for the atrium.

12. A cardiac pacemaker as claimed in any one of the preceding Claims, characterised in that in the event of signals picked up substantially simultaneously in the atrium and ventricle, those in the ventricle have priority and the signals in question are processed as originating from the ventricle.

13. A cardiac pacemaker as claimed in Claim 12, characterised in that a delay element (2) is provided for the signals picked up in the atrium.

14. A cardiac pacemaker as claimed in any one of the preceding Claims, characterised in that programming means (29 to 31) are provided to alter the parameters of the pacemaker and that the delay periods of the first and second signal transmitting means can each be adjusted, via programming means, to a value valid for normal operation without tacharrhythmias.

15. A cardiac pacemaker as claimed in Claim 14, characterised in that the shortened delay periods can also be preset by the programming means, which periods only become effective on activation of the first and second timing means.

**Revendications**

1. Stimulateur cardiaque avec des moyens (6, 16) pour stimuler l'oreillette respectivement le ventricule à des intervalles de temps préfixés à la suite d'un signal recueilli dans l'autre de ces deux cavités du cœur, ou d'une impulsion de stimulation délivrée à l'autre cavité, comprenant : des premiers moyens d'horloge (9) qui, à la suite d'un signal recueilli dans l'oreillette, déclenchent une impulsion de stimulation dans le ventricule après une temporisation qui est raccourcie par rapport au temps de transition auriculo-ventriculaire physiologique, ainsi que des seconds moyens d'horloge (8) qui, à la suite d'un signal recueilli dans le ventricule, déclenchent une impulsion de stimulation dans l'oreillette après une temporisation qui est raccourcie par rapport à l'intervalle de temps ventriculo-auriculaire physiologique, caractérisé en ce que, pour mettre fin à des tachy-arythmies, il est pourvu de détecteurs (35, 34) pour détecter des activités sinusales prématurées et/ou des extrasystoles, et qui délivrent des signaux de sortie pour actionner les premiers et seconds moyens d'horloge.

2. Stimulateur selon la revendication 1, caractérisé en ce que le détecteur (35) pour les activités sinusales prématurées, qui sert à actionner les premiers moyens d'horloge, est un détecteur destiné à établir l'intervalle entre des ondes P successives provenant de l'oreillette.

3. Stimulateur selon une des revendications précédentes, caractérisé en ce que les seconds moyens d'horloge (8) peuvent être actionnés par le détecteur (35) pour les extrasystoles.

4. Stimulateur selon la revendication 3, caractérisé en ce que la temporisation raccourcie des seconds moyens d'horloge est ajustable à une valeur minimale dans le cas d'une première extrasystole et peut être augmentée d'une valeur, susceptible d'être fixée d'avance, à chaque extrasystole consécutive.

5. Stimulateur selon la revendication 3 ou 4, caractérisé en ce que le détecteur pour les extrasystoles est un compteur (34).

6. Stimulateur selon la revendication 5, caractérisé en ce que le compteur (34) est susceptible d'être remis à sa position initiale par un signal provenant de l'oreillette.

7. Stimulateur selon une des revendications précédentes, caractérisé en ce qu'il comprend au moins un élément réfractaire ou de blocage (7, 14) qui, à la suite d'un signal recueilli dans l'oreillette et/ou le ventricule, empêche, pendant un laps de temps préfixé, la retransmission de signaux consécutifs recueillis dans l'oreillette respectivement le ventricule.

8. Stimulateur selon la revendication 7, caractérisé en ce que l'élément réfractaire (7) est réalisé de manière que la période réfractaire, dans le cas d'un signal recueilli dans l'oreillette, peut être superposée à la période réfractaire dans le cas d'un signal recueilli dans le ventricule, de manière que la période réfractaire effective pour les signaux recueillis dans le ventricule soit prolongée du temps de transition auriculo-ventriculaire physiologique.

9. Stimulateur selon une des revendications précédentes, caractérisé en ce qu'il comprend au moins un élément réfractaire ou de blocage (17) qui est sensible à des signaux de sortie du stimulateur et qui, après chaque impulsion de sortie, bloque la sortie concernée et/ou l'entrée de l'amplificateur de sortie correspondant pendant un laps de temps prédéterminé.

10. Stimulateur selon une des revendications précédentes, caractérisé en ce que les seconds moyens d'horloge (8) sont suivis d'une horloge (5) qui est réalisée de manière à déclencher automatiquement des impulsions de stimulation pour l'oreillette lorsque, pendant un laps de temps prédéterminé, elle ne reçoit pas de signal provenant des seconds moyens d'horloge ou de signal provenant de l'électrode (A) placée dans l'oreillette.

11. Stimulateur selon une des revendications précédentes, caractérisé en ce que le ventricule est susceptible d'être stimulé, alors que les premiers moyens d'horloge (9) ne sont pas actionnés, avec un retard, par rapport à un signal provenant de l'oreillette et/ou d'une impulsion de stimulation pour l'oreillette, qui correspond au temps de transition auriculo-ventriculaire physiologique.

12. Stimulateur selon une des revendications précédentes, caractérisé en ce que, au cas où des signaux sont recueillis sensiblement en même temps dans l'oreillette et le ventricule, les signaux recueillis dans le ventricule sont prioritaires et les signaux en question sont traités comme des signaux provenant du ventricule.

13. Stimulateur selon la revendication 12, caractérisé en ce qu'il comprend un élément de temporisation (12) pour les signaux recueillis dans l'oreillette.

14. Stimulateur selon une des revendications précédentes, caractérisé en ce qu'il comprend des moyens de programmation (29 à 31) pour changer les paramètres du stimulateur et que les temporisations des premiers et seconds moyens d'horloge peuvent être ajustées, par les moyens de programmation, à une valeur convenant à un fonctionnement normal sans tachy-arythmies.

15. Stimulateur selon la revendication 14, caractérisé en ce que les moyens de programmation sont également capables de préfixer les temporisations raccourcies, devenant seulement effectives en cas d'actionnement des premiers et seconds moyens d'horloge.

0 063 097

FIG.1

FIG.2

FIG.3a

FIG.3b

FIG.3c

FIG.3d

FIG.3e

FIG.3f

FIG.3g

FIG.4

FIG.5

FIG.6

FIG.7

FIG.8